# EUROPEAN PATENT SPECIFICATION

(11) **EP 1 619 501 B1**
(45) Date of publication and mention of the grant of the patent: **26.09.2007**
(21) Application number: 04017375.9
(22) Date of filing: 22.07.2004
(51) Int. Cl.: G01N 33/533, A61K 49/00

(54) **Use of cyanine dyes for the diagnosis of disease associated with angiogenesis**
Verwendung von Cyanin-farbstoffen zur Diagnose von Krankheiten, welche mit Angiogenese assoziert sind
Utilisation des colorants cyanines dans le diagnostic des maladies associées à l'angiogénèse

(43) Date of publication of application: 25.01.2006
(60) Divisional application: 07090023.8
(73) Proprietor: Bayer Schering Pharma Aktiengesellschaft, 13353 Berlin (DE)
(72) Inventor: Schirner, Michael, 13158 Berlin (DE); Licha, Kai, 14612 Falkensee (DE); Hauff, Peter, 10439 Berlin (DE); Perlitz, Christin, 10115 Berlin (DE)
(74) Representative: Kilger, Ute

(56) References cited:
- EP-A- 0 286 252
- WO-A-01/23005
- WO-A-20/04065491
- WO-A1-01/62800
- WO-A2-03/076469
- US-A1- 2003 143 159
- US-A1- 2004 001 790
- SCHEUERMANN JÖRG: "Isolation of binding molecules to the EDB domain of fibronectin, a marker of angiogenesis" 2002, SWISS FEDERAL INSTITUTE OF TECHNOLOGY, ZURICH: DISS. ETH NR. 14959 , HTTP://E-COLLECTION.ETHBIB.ETHZ.CH/SHOW?TY PE=DISS&NR=14959 , XP002314651 pages 7-8: Zusammenfassung

## Description

This invention relates to the use of conjugates of cyanine dyes with an angiogenesis specific binding component preferably with an EB-D fibronectin specific binding component for the diagnosis of micrometastasis and small proliferative lesions, in particular primary tumors, precancerosis, dysplasia, metaplasia, endometriosis, ocular diseases associated with angiogenesis, psoriasis, psoriatic arthritis and/or rheumatoid arthritis.

### Background of the Invention

The use of light in medical diagnosis has recently gained importance (see, e.g., Biomedical Photonics Handbook (Editor: T. Vo-Dinh), CRC Press). A wide variety of diagnostic processes are under experimental testing for application in various medical disciplines, e.g. endoscopy, mammography, surgery or gynecology. To this end dyes are fed to the tissue as exogenic contrast media for fluorescence diagnosis and imaging, and here in particular fluorescence dyes with an absorption and fluorescence maximum in the spectral range of 700-900 nm (diagnostic window of tissue), have been used for *in vivo* imaging. Photons of this wavelength are comparatively little absorbed by tissue and can therefore penetrate several centimeters into the tissue before the absorption process (primarily by oxyhemoglobin and deoxyhemoglobin) ends the light transport. Absorption can take place, moreover, by the fluorescence dyes that are introduced into the tissue, but that emit the absorbed energy in the form of longer-wave fluorescence radiation. This fluorescence radiation can be detected spectrally separated and makes possible the localization of dyes and the correlation with molecular structures to which the dye has bonded (see in this respect also Licha, K. (2002) Contrast Agents for Optical Imaging (Review). In: Topics in Current Chemistry - Contrast Agents II (Editor: W. Krause), Volume 222, Springer Heidelberg, pp. 1 - 31.).

Fluorescence dyes from the class of cyanine dyes fall into the category of promising representatives and were synthesized in many different structural widths. In particular, carbocyanines with indocarbocyanine, indodicarbocyanine and indotricarbocyanine skeletons have high extinction coefficients and good fluorescence quantum yields (Licha, K. (2002) *supra,* and the references cited therein).

To achieve a diagnostically significant differentiation between diseased structures and healthy tissue, the dye that is administered must lead to as high a concentration difference between the two tissue types as possible. This can be carried out based on tumor-physiological or morphological properties (blood supply, distribution kinetics, delayed removal, vessel structures) as well as based on molecular properties of the tumor and vessel cell or adjacent tissue. For molecular labeling of disease-specific structures, conjugates that consist of fluorescence dyes with target-affine molecules, such as proteins, peptides, or antibodies, can be used. After injection, a certain portion of these conjugates binds to molecular target structures, such as receptors, cell surface structures or matrix proteins, while the unbonded portion remains diluted or metabolized in the bodily fluids or is excreted from the body. In this way, a higher concentration difference and, thus, a greater image contrast in implementing the fluorescence diagnosis may result (high signal-to-noise ratio).

It has been described that many diseases like, for example, tumors (Folkman J. (1974). Symp. Soc. Dev. Biol. 30:43-52), arthritis (Colville-Nash PR, Scott DL (1992) Ann. Rheum. Dis. 51:919-25), psoriasis (Folkman J. (1972) J. Invest. Dermatol. 59:40-43), ocular diseases (Adamis AP, et al. (1999) Angiogenesis, 3:9-14) are associated with angiogenesis. The various diseases associated with angiogenesis are reviewed in, for example, Longo R, et al. (2002) Angiogenesis 5:237-56. On the other hand the formation of new blood vessels rarely occurs in healthy tissue with a few exceptions including wound healing and the changes in endometrial tissue during the menstrual cycle or pregnancy. Thus, neoangiogenesis has become both an important therapeutic as well as diagnostic target.

Many molecular structures that are preferentially or exclusively present in or in the vicinity of growing vascular cells have been described (for a review see, for example, Alessi P, et al. (2004) Biochim. Biophys. Acta. 1654:39-49 and Nanda A and St. Croix B (2004) Curr. Opin. Oncol. 16:44-49) including receptors on the endothelial cells like vascular endothelial growth factor receptor (VEGF-R) and matrix proteins like extra domain B (ED-B) fibronectin. The ED-B domain of fibronectin, a sequence of 91 amino acids identical in mouse, rat and human, which is inserted by alternative splicing into the fibronectin molecule, has been shown to specifically accumulate around neo-vascular structures (Castellani et al. (1994). Int. J. Cancer 59:612-618).

A micrometastasis is a cohesive cluster of malignant cells > 0.2 mm and a cluster of malignant cells < 0.2 mm is called sub-micrometastasis (Van der Westhuizen N. (2002) Laboratory Report; Rampaul RS, et al. (2001) Breast Cancer Res. 3:113-116; Bitterman A., et al. (2002) IMAJ 4:803-809). Micrometastasis which presently can only be detected *in vitro* with a microscope can be angiogenic or non-angiogenic. Most human tumors including primary tumors and metastasis arise without angiogenic activity and exist *in situ* as a microscopic lesion of 0.2 to < 2 mm in diameter for months to years, after which a small percentage may switch to the angiogenic phenotype (Folkman J and Becker K (2000) Acad. Radiol. 7:783-785; Folkman J (2001) Angiogenesis. In Braunwald E, et al., Harrison's Textbook of Internal Medicine, 15th Edition, McGraw-Hill, 517-530). At the cellular level at least four mechanisms of the angiogenic switch have been identified in human and mouse tumors: (1) avascular *in situ* carcinoma can recruit their own blood supply by stimulating neovascularization in an adjacent host vascular bed - the most common process in human tumors, (2) circulating precursor endothelial cells from bone marrow may incorporate into an angiogenic focus, (3) tumors may induce host fibroblast and/or macrophages in the tumor bed to overexpress an angiogenic factor (e.g. , vascular endothelial growth factor (VEGF)); and (4) preexisting vessels can be coopted by tumor cells. The angiogenic switch may also include combinations of these mechanisms (Folkman J (2001) Angiogenesis. In Braunwald E, et al., Harrison's Textbook of Internal Medicine, 15th Edition, McGraw-Hill, 517-530). It is now widely accepted that the "angiogenic switch" is "off" when the effect of pro-angiogenic molecules is balanced by that of anti-angiogenic molecules, and is "on" when the net balance is tipped in favour of angiogenesis. Various signals that trigger this switch have been discovered. Angiogenesis activators are molecular structures as e.g., VEGF family members, VEGFR, NRP-1, Ang1, Thie2, PDGF-BB and receptors, TGF-β1, endoglin, TGF-B receptors, FGF, HGF, MCP-1, Integrins (αᵥβ₃, αᵥβ₅, α₅β₁), VE-cadherin, PECAM (CD31), Ephrins, Plasminogen activators, MMPs, PAI-1, NOS, COX-2, AC133, Chemokins or Id1/Id3. Angiogenesis inhibitors are molecular structures as e.g., VEGFR-1, Ang2, TSP-1,-2, Angiostatin and related plasminogen kringles, Endostatin (collagen XVII fragment), Vasostatin, Platelet factor 4, TIMPs, MMP inhibitors, PEX, Meth-1, Meth-2, IFN-α, -β, -γ, IP-10, IL-4, IL-12, IL-18, Prolactin (*M*, 16K), VEGI, Fragment of SPARC, Osteopontin fragment or Maspin (Carmeliet P and Jain RK. (2000) Nature 407:249-257; Yancopoulos GD et al. (2000) Nature 407:242-248; Bergers G. and Benjamin LE (2002) Nature Reviews Cancer 3:401-410; Hendrix MJC et al. (2002) Nature Reviews Cancer 3:411-421).

Ntziachristos V, et al. (2000) Proc. Natl. Acad. Sci. U.S.A. 97:2767-2772 describe diffuse optical tomography of fibroadenoma with indocyanine green enhancement. The resolved tumors are primary tumors with a size in excess of 1 cm.

WO 01/23005 A1 describes conjugates of ED-B specific antibodies and various dyes, and their use in the delineation of tumor peripheries. No teaching is provided on the spatial resolution that can be obtained with these conjugates.

McDonald D.M and Choyke P.L (2003) Nat. Med. 9: 713-25 review advances in imaging of angiogenesis. They discuss how magnetic resonance imaging (MRI), computer tomography (CT), positron emission tomography (PET), ultrasonography and optical imaging provide noninvasive methods to obtain images of angiogenesis in animals and humans. They teach that these methods provide their highest resolution on preserved tissue specimen, whereas clinical methods give images of living tissues at much lower resolution and specificity and can not resolve vessels of the microcirculation. It concludes that future challenges include developing new imaging methods that can bridge this resolution gap and specifically identify angiogenic vessels. Presently, no such methods are available.

### Detailed Description of the Invention

Given the difficulties in the prior art to image micrometastasis and newly vascularized or vascularizing structures, i.e. structures, which comprise primarily microvasculature or which are in the process of developing a microvasculature, it has been surprisingly found by the present inventors that such structures can be distinguished by light based diagnosis using conjugates of an angiogenesis specific binding component, in particular ED-B fibronectin specific binding components, and cyanine dye(s). This observation opens the use of near infrared fluorescent imaging to new fields of diagnosis, which require the detection of small diseased structures. Therefore, in a first aspect the present invention provides the use of a conjugate of the general formula (I):

B-(D)ₙ (I),

wherein
- B: stands for an angiogenesis specific binding component,
- D: stands for a cyanine dye, and
- n: is 1 to 5
for the production of a diagnostic for the diagnosis of micrometastasis and small proliferative lesions.

The angiogenesis specific binding component binds to structures, which are preferentially or exclusively present in micrometastasis, in or in the vicinity of newly formed microvessels or which are present prior or during growth of microvascular structures. Such molecular structures are reviewed in, for example, WO 96/01653, Alessi P, et al. (2004) and Nanda A and St Croix B. (2004). As pointed out above cells forming a micrometastasis and similarly cells of small proliferative lesions express both angiogenic and antiangiogenic factors; which as long as the angiogenesis inhibitors counteract the effect of the angiogenic factors leads to a suppression of angiogenesis. Once the effect of the angiogenic factors prevail they lead to initiation of angiogenesis. Angiogenesis activators include without limitation molecular structures like, e.g. ED-B fibronectin (ED-BF), endoglin (CD105) (Burrows FJ et al. (1995) Clin. Cancer Res.. 1: 1623-1634), VEGF family members, vascular endothelial growth factor (VEGFR), NRP-1, Ang1, Thie2, PDGF-BB and receptors, TGF-β1, TGF-β receptors, FGF, HGF, MCP-1, Integrins (αᵥβ₃, αᵥβ₅, α₅β₁), VE-cadherin, PECAM (CD31), Ephrins, Plasminogen activators, MMPs, PAI-1, NOS, COX-2, AC133, chemokines or Id1/Id3. Angiogenesis inhibitors include without limitation molecular structures like, e.g. VEGFR-1, Ang2, TSP-1,-2, Angiostatin and related plasminogen kringles, Endostatin (collagen XVII fragment), Vasostatin, Platelet factor 4, TIMPs, MMP inhibitors, PEX, Meth-1, Meth-2, IFN-α, -β, -γ, IP-10, IL-4, IL-12, IL-18, Prolactin (*M*, 16K), VEGI, Fragment of SPARC, Osteopontin fragment or Maspin (Carmeliet P and Jain RK (2000) Nature 407:249-257; Yancopoulos GD et al. (2000) Nature 407:242-248; Bergers G and Benjamin LE (2002) Nature Reviews Cancer 3:401-410; Hendrix MJC et al. (2002) Nature Reviews Cancer 3:411-421). In a preferred embodiment the angiogenesis specific binding component is ED-BF. Out of those ED-BF is a particular preferred target structure. ED-BF is splice variant of fibronectin also called oncofoetal fibronectin, which is specifically formed in newly grown microvascular structures during angiogenesis.

The component that binds to these structures is preferably a peptide (amino acid chain with two to 50 amino acid residues), a protein (amino acid chains with more than 50 amino acid residues), a nucleic acid, a small molecule, or a sugar.

Preferred proteins or peptides are ligands of receptors, which are preferentially or exclusively expressed in micrometastasis and/or nearly vascularized or vascularizing structures, in particular vascular endothelial growth factor (VEGF), and antibodies, including human, humanized and chimeric antibodies; antibody binding domain comprising fragments, e.g. Fv, Fab, Fab', F(ab')₂, Fabc, Facb; single chain antibodies, e.g. single chain Fvs (scFvs); and diabodies.

A large variety of such antibodies has been described in the literature and include for ED-BF L19 and E8 (see Viti F. et al. (1999) Cancer Res. 59:347-352), the BC-1 monoclonal antibody described in EP 0 344 134 B1, which is obtainable from the hybridoma deposited at the European Collection of Animal Cell Cultures, Porton Down, Salisbury, UK under the number 88042101 or a chimeric or humanized version thereof, the antibodies against ED-BF with the specific V_{L} and V_{H} sequences disclosed in WO 97/45544 A1, the antibodies against ED-BF with the specific V_{L} and V_{H} sequences disclosed in WO 99/5857 A2, the antibodies against ED-BF with the specific V_{L} and V_{H} sequences disclosed in WO 01/62800 A1 and AP38 and AP39 (Marty C, et al. (2001) Protein Expr. Purif. 21:156-64). Antibodies specific to ED-BF have been reviewed in Ebbinghaus C, et al. (2004) Curr Pharm Des. 10:1537-49. All these antibodies or antibody binding fragments thereof can be used as angiogenesis specific binding component in a preferred use of the present invention. Particularly preferred antibodies are L19, E8, AP 38 and AP 39 or binding domain comprising fragments thereof.

It is well known in the art that nucleic acids can possess specific binding properties, thus, the angiogenesis specific binding component can also be a nucleic acid. Preferably, such nucleic acids include DNA, RNA, aptamers, and PNA, wherein aptamers are particularly preferred. Methods to identify specifically binding aptamers are well known in the art and are described, for example, in WO 93/24508 A1, WO 94/08050 A1, WO 95/07364 A1, WO 96/27605 A1, and WO 96/34875 A1. The methods disclosed in these documents are hereby specifically referenced and can be used in the identification of angiogenesis specific binding aptamers useable in the present invention. Preferred aptamers employed in the use of the present invention specifically recognize ED-BF.

With the advent of high throughput screening of small molecules, i.e. non peptidly, non nucleic acid compounds, of a molecular weight lower than 1.000 g/mol, preferably lower than 500 g/mol, it has been possible to identify small molecules with particular binding properties. Such small molecules can equally be employed as one component of the conjugate usable according to the present invention. A preferred small molecule is 2,2-diphenylethylamine, which has been identified to specifically bind to ED-BF (Scheuermann J. (2002) Isolation of binding molecules to the EDB domain of fibronectin, a marker of angiogenesis. Dissertation submitted to Swiss Federal Inst. of Technology, Zurich).

In a preferred use of the present invention the cyanine dye is selected from the group consisting of carbocyanine, dicarbocyanine, and tricarbocyanine. The synthesis of cyanine dyes useable according to the present invention can be carried out using the methods known in the state of the art and which are exemplified in, e.g. Hamer F.M. The Cyanine Dyes and Related Compounds, John Wiley and Sons, New York 1964; Ernst LA, et al. (1989) Cytometry 10:3-10; Southwick PL, et al., (1990) Cytometry 11:418-430; Lansdorp PM et al., (1991) Cytometry 12:723-730; Mujumdor RB et al., (1993) Bioconjugate Chem. 4:105-11; Mujumdor SR et al., (1996) Bioconjugate Chem. 7:356-62; Flanagan JH et al., (1997) Bioconjugate Chem. 8:751-56; Keil D et al., (1991) Dyes and Pigments 17:19-27; Terpetschnig E and Lakowicz JR (1993) Dyes and Pigments 21:227-34; Terpetschnig E et al., (1994) Anal. Biochem. 217: 197-204; Lindsey JS et al., (1989) Tetrahedron 45:4845-66; Górecki T et al., (1996) J. Heterocycl. Chem. 33, 1871-6; Narayanan N and Patonay G (1995) J. Org. Chem. 60:2391-5, 1995; and Terpetschnig E et al., (1993) J. Fluoresc. 3:153-155. Additional processes are described in patent publications US 4,981,977; US 5,688,966; US 5,808,044; EP 0 591 820 Al; WO 97/42976; WO 97/42978; WO 98/22146; WO 98/26077; and EP 0800831.

Moreover, indotricarbocyanines with altered substituents were synthesized and coupled to biomolecules (described in, e.g. Becker A et al., Photochem. Photobiol. 72, 234, 2000; Licha K et al., Bioconjugate Chem. 12, 44, 2001; Becker A et al., Nature Biotechnol. 19, 327, 2001; Bugaj JE et al., J. Biomed. Optics 6, 122, 2001;Achilefu S et al., J. Med. Chem. 45, 2003, 2002). Other examples are found in particular in the publications WO 00/61194 ("Short-Chain Peptide Dye Conjugates as Contrast Agents for Optical Diagnostics"), WO 00/71162, WO 01/52746, WO 01/52743 and WO 01/62156. Another process for the production of an indotricarbocyanine dye is a simple access via 4-substituted pyridines. Various 4-substituted pyridines can be converted by means of the Zincke reaction (Zincke-König reaction, see Römpps Chemie Lexikon [Römpps Chemical Dictionary], 10th Edition, page 5067) in high yields into meso-substituted glutaconaldehyde-dianilide, which are precursors to cyanine dyes.

The especially preferred indotricarbocyanine dye, which can be used according to the invention is the one with formula XXVII in Table 1 below showing the structure of the dye prior to coupling to B and comprising either a maleinimide (maleimide) or bromoacetyl coupling moiety, which facilitates coupling to thiol-group containing angiogenesis specific binding components. In the resulting conjugates the respective coupling moiety will in some embodiments not be present anymore, if it was a leaving group, or only parts of it will remain in the conjugate called residual part of a coupling moiety. It will be apparent to someone of skill in the art that other moieties instead of the maleinimide (maleimide) or bromoacetyl coupling moieties depicted below can be substitued in below structures, including, for example, chloroacetyl, iodoacetyl, chloroacetamido, bromoacetamido, iodoacetamido, chloroalkyl, bromoalkyl, iodoalkyl, pyridyl disulfide and vinyl sulfonamide, to effect coupling reactions to B.

**Table 1**

| Preferred dye which can be used for coupling to an angiogenesis specific binding component: | |
|---|---|
| (XXVII) Example 10 | 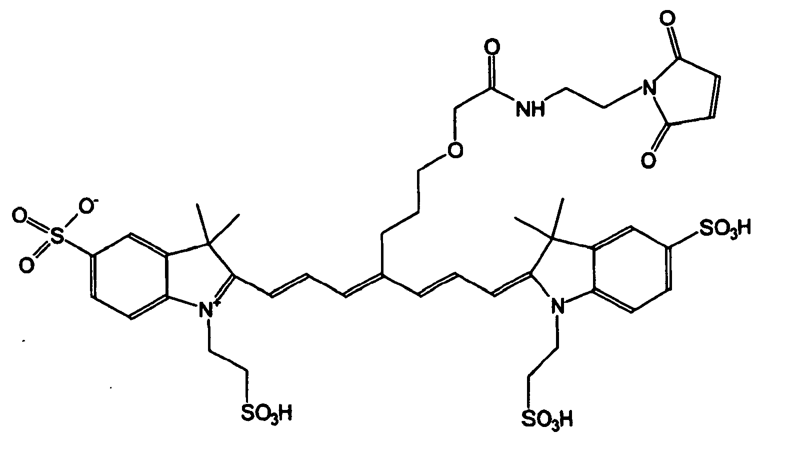 |

The appropriate method for coupling the respective cyanine dye and the respective angiogenesis specific binding component primarily depends on the chemical nature of the angiogenesis specific binding component. A large variety of residues or groups are known in the art, which are naturally present or can be introduced into the various angiogenesis specific binding components, e.g. -NH₂, -COOH, -SH, -OH etc.. These groups can then form covalent bonds with groups attached to the cyanine dyes, which show a good reactivity, towards the other group resulting in the coupling of the two components. Of course it is also possible to inverse the order, i.e. attach the reactable group to the cyanine dye and the reactive group to the angiogenesis specific binding component. Based on this teaching the skilled person is able to choose appropriate reactive and reactable groups for each respective pair of a cyanine dye and an angiogenesis specific binding component.

Many proteins or peptides comprise thiol-groups, e.g. of cysteine residues, or can be modified to comprise thiol groups. Therefore, if the conjugate used in the present invention comprises a peptide or protein and (a) cyanine dye(s) it is preferred that the cyanine dyes mentioned above comprise prior to coupling to the protein or peptide a thiol group-reactive coupling moiety. Thiol group-reactive functionalities are well known in the art and comprise, e.g. maleinimide (maleimide), chloroacetyl, bromoacetyl, iodoacetyl, chloroacetamido, bromoacetamido, iodoacetamido, chloroalkyl, bromoalkyl, iodoalkyl, pyridyl disulfide and vinyl sulfonamide. Thus, in a preferred embodiment R³ or R^{10'} in above embodiments represented such a coupling moiety prior to linkage to B. If R³ is a linker connected to B the linker comprises such coupling moiety prior to coupling.

For some coupling moieties, which are not entirely replaced during the coupling reaction, e.g. which are not a leaving group in the coupling reaction, a part of the coupling moiety may remain attached to R³, to the linker or to R^{10'}. This part, which may remain at the junction of the cyanine dye and the angiogenesis specific binding component is referred to as "residual part of the coupling moiety".

The pharmaceutically acceptable salt may be any as long as it forms a non-toxic salt with the cyanine compounds outlined above. Examples include alkali metal salts such as sodium salts, potassium salts; salts of alkaline earth metals such as magnesium salts, calcium salts and the like organic ammonium salts such as triethyl ammonium salts, tributyl ammonium salts, pyridinium salts and the like, salts of amino acids such as lysine, arginine and the like. Preferred salts are sodium salts.

Small proliferative lesions are in a preferred embodiment a primary tumor, a precancerosis, a dysplasia, a metaplasia, psoriasis, psoriatic arthritis, rheumatoid arthritis, endometriosis and/or ocular diseases associated with angiogenesis. In a preferred use of the present invention the conjugate(s) is (are) used for *in vivo* diagnosis of the above indicated diseases and/or micrometastasis.

The use of the present invention can be for routine diagnosis, i.e. for screening for the respectively indicated diseases. However, in a preferred embodiment the conjugates are used once a disease has been diagnosed with, for example, a standard x-ray procedure, e.g. mammography, a whole body scans or MRI. The patient is then examined for further micrometastasis and/or small (additional) primary tumor(s). Such an examination can occur for a better assessment of the severity, e.g. stage of a disease, in order to determine the best treatment options and/or prior, during and/or after a treatment procedure (e.g., drugs, radiation or surgery). If performed prior to a treatment procedure the use of the diagnostic of the present invention allows the determination whether, e.g. micrometastases have already formed in the vicinity of the primary tumor and, thus, whether a lumpectomy or rather a mastectomy is indicated as an example in breast cancer. After treatment the use of the diagnostic of the present invention allows to assess the success of the treatment procedure and to determine subsequent treatment regimens, e.g. radiation or chemotherapy. When used during a surgical procedure it is, for example, possible to detect micrometastasis in tissue, e.g. lymph nodes, surrounding the primary tumor. In this embodiment the use of the present invention allows a more complete removal of tumors or micrometastasis during the procedure.

The use according to the present invention allows the detection of events preceding the onset of angiogenesis, the onset of angiogenesis or angiogenesis, i.e. already formed microvasculature, even when it occurs in very small tissue structures. In a preferred embodiment of the present invention the micrometastasis and/or the small proliferative lesions, in particular the micrometastasis, the precancerosis, the dysplasia, the metaplasia, endometriosis and/or the primary tumor(s), which are detected with the present invention has (have) a diameter of less than 10 mm, preferably of less than 8 mm, more preferably of less than 6 mm, more preferably of less than 5 mm, more preferably of less than 4 mm, more preferably of less than 3 mm, more preferably of less than 2 mm and most preferably of less than 1 mm. A particular preferred range of the micrometastasis and/or the small proliferative lesions, in particular the micrometastasis, the precancerosis, the dysplasia, the metaplasia, endometriosis and/or the primary tumor(s), detectable according to the use of the present invention are between about 10 mm to about 0.2 mm, more preferably between about 8 mm to about 0.2 mm, more preferably between about 6 mm to about 0.2 mm, more preferably between about 5 mm to about 0.2 mm, more preferably between about 4 mm to about 0.2 mm, more preferably between about 3 mm to about 0.2 mm and most preferably between about 2 mm to about 0.2 mm.

Preferably the micrometastasis, which is detected according to the use of the present invention is an iatrogenic micrometastasis, a hematogenous micrometastasis, a cavitary micrometastasis, an intraluminal micrometastasis, a lymphatic micrometastasis, a local micrometastasis, and/or a regional micrometastasis.

The micrometastasis diagnosed preferably originates from a primary tumor including but not limited to malignomas (e.g., carcinomas, sarcomas) of the gastrointestinal or colorectal tract, liver, pancreas, kidney, bladder, prostate, endometrium, ovary, testes, melanoma, dysplastic oral mucosa, invasive oral cancers, small cell and non-small cell lung carcinomas; mammary tumors, e.g. a hormone-dependent breast cancers, hormone independent breast cancers; transitional and squamous cell cancers; neurological malignancies including neuroblastoma, gliomas, astrocytomas, osteosarcomas; soft tissue sarcomas; hemangioamas and endocrinological tumors. The small primary tumor detectable according to the use of the present invention preferably is one of the above indicated tumors. In a particular preferred embodiment the small primary tumor or the micrometastasis is a mammary tumor, in particular a hormone-dependent breast cancer or hormone independent breast cancer.

The precancerosis, which is detectable according to the use of the present invention is preferably selected from the group consisting of precancerosis of the skin, in particular actinic keratosis, cutaneaous horn, actinic cheilitis, tar keratosis, arsenic keratosis, x-ray keratosis, Bowen's disease, bowenoid papulosis, lentigo maligna, lichen sclerosus, and lichen rubber mucosae; precancerosis of the digestive tract, in particular erythroplakia, leukoplakia, Barrett's esophagus, Plummer-Vinson syndrome, crural ulcer, gastropathia hypertrophica gigantea, borderline carcinoma, neoplastic intestinal polyp, rectal polyp, porcelain gallbladder; gynaecological precancerosis, in particular carcinoma ductale in situ (CDIS), cervical intraepithelial neoplasia (CIN), leukoplakia, endometrial hyperplasia (grade III), vulvar dystrophy, vulvar intraepithelial neoplasia (VIN), hydatidiform mole; urologic precancerosis, in particular bladder papillomatosis, Queyrat's erythroplasia, testicular intraepithelial neoplasia (TIN), leukoplakia; carcinoma in situ (CIS); precancerosis caused by chronic inflammation, in particular pyoderma, osteomyelitis, acne conglobata, lupus vulgaris, and fistula.

Dysplasia is frequently a forerunner of cancer, and is found mainly in the epithelia; it is the most disorderly form of non-neoplastic cell growth, involving a loss in individual cell uniformity and in the architectural orientation of cells. Dysplastic cells often have abnormally large, deeply stained nuclei, and exhibit pleomorphism. Dysplasia characteristically occurs where there exist chronic irritation or inflammation. Dysplastic disorders which can be diagnosed according to the present invention include, but are not limited to, anhidrotic ectodermal dysplasia, anterofacial dysplasia, asphyxiating thoracic dysplasia, atriodigital dysplasia, bronchopulmonary dysplasia, cerebral dysplasia, cervical dysplasia, chondroectodermal dysplasia, cleidocranial dysplasia, congenital ectodermal dysplasia, craniodiaphysial dysplasia, craniocarpotarsal dysplasia, craniometaphysial dysplasia, dentin dysplasia, diaphysial dysplasia, ectodermal dysplasia, enamel dysplasia, encephalo-ophthalmic dysplasia, dysplasia epiphysialis heminelia, dysplasia epiphysialis multiplex, dysplasia epiphysalis punctata, epithelial dysplasia, faciodigitogenital dysplasia, familial fibrous dysplasia of jaws, familial white folded dysplasia, fibromuscular dysplasia, fibrous dysplasia of bone, florid osseous dysplasia, hereditary renal-retinal dysplasia hidrotic ectodermal dysplasia, hypohidrotic ectodermal dysplasia, lymphopenic thymic dysplasia, mammary dysplasia, mandibulofacial dysplasia, metaphysical dysplasia, Mondini dysplasia, monostotic fibrous dysplasia, mucoepithelial dysplasia, multiple epiphysial dysplasia, oculoauriculovertebral dysplasia, oculodentodigital dysplasia, oculovertebral dysplasia, odontogenic dysplasia, ophthalmomandibulomelic dysplasia, periapical cemental dysplasia, polyostotic fibrous dysplasia, pseudoachondroplastic spondyloepiphysial dysplasia, retinal dysplasia, septo-optic dysplasia, spondyloepiphysial dysplasia, and ventriculoradial dysplasia.

Metaplasia is a form of controlled cell growth in which one type of adult or fully differentiated cell substitutes for another type of adult cell. Metaplastic disorders, which are detectable according to the use of the present invention are preferably selected from the group consisting of agnogenic myeloid metaplasia, apocrine metaplasia, atypical metaplasia, autoparenchymatous metaplasia, connective tissue metaplasia, epithelial metaplasia, intestinal metaplasia, metaplastic anemia, metaplastic ossification, metaplastic polyps, myeloid metaplasia, primary myeloid metaplasia, secondary myeloid metaplasia, squamous metaplasia, squamous metaplasia of amnion, symptomatic myeloid metaplasia and regenerative metaplasia.

The ocular disease, which is detectable according to the use of the present invention is preferably selected from the group consisting of trachoma, retinopathy of prematurity, diabetic retinopathy, neovascular glaucoma and age-related macular degeneration.

Endometriosis is a gynecological disease defined by the proliferation of endometrial tissue outside the uterine cavity. Proliferating endometrial cells can distribute through the entire body and endometrial lesions have already been found in the lung and in other organs and in that respect the distribution of endometrial lesions resembles the distribution of micrometastasis. In a preferred embodiment of the use of the present invention the endometric lesions, e.g. endometrial cell clusters, which are detected are hematogenous cell clusters, cavitary cell clusters, intraluminal cell clusters, lymphatic cell clusters, local cell clusters and/or regional cell clusters.

The dose of the conjugat is not particularly limited insofar as the dose enables detection of the site to be ultimately diagnosed. It is appropriately adjusted depending on the kind of compound to be used, age, body weight and target organ or tissue and the like. Typically the dose is between 0.002 to 100 mg/kg body weight, preferably between 0.005 to 10 mg/kg body weight, more preferably between 0.01 to 2 mg/kg body weight, and most preferably between 0.02 to 1 mg/kg body weight.

The fluorescence imaging method of the present invention is practised following known methods, and each parameter, such as excitation wavelength and fluorescence wavelength to be detected, can appropriately be determined for each conjugate to be administered, to achieve optimal imaging and resolution. The time spend from administration of the conjugates to the determination by the fluorescence imaging method varies depending on the conjugate and the administration target. For example, when the conjugate is used for tumor imaging the lapse time typically will be in the range of about 2 to 120 hours after administration and preferably between about 2 to about 10 h after administration. When the lapse time is too short the fluorescence is so intense that angiogenic and non-angiogenic tissues can not be clearly differentiated (low signal-to-noise ratio). Devices for the fluorescence imaging method are well known in the art and are describe in, for example, EP 0 868 143, EP 1 146 811 A1, EP 1 408 824 A2, EP 1 409 995 A1, and EP 1 410 330 A2.

As has been outlined above the present invention can also be used in connection with surgical procedures and, therefore, the detection of the fluorescence can be carried out using surgical microscopes, microscopes, magnifying glasses and the like. Such devices can be employed both in a variety of surgical procedures including open and endoscopic procedures. It is also possible to use the invention in connection with devices and procedures, which are commonly used for routine screening for cancers, e.g. colonoscopy and gastroscopy.

### Brief Description of the Figure

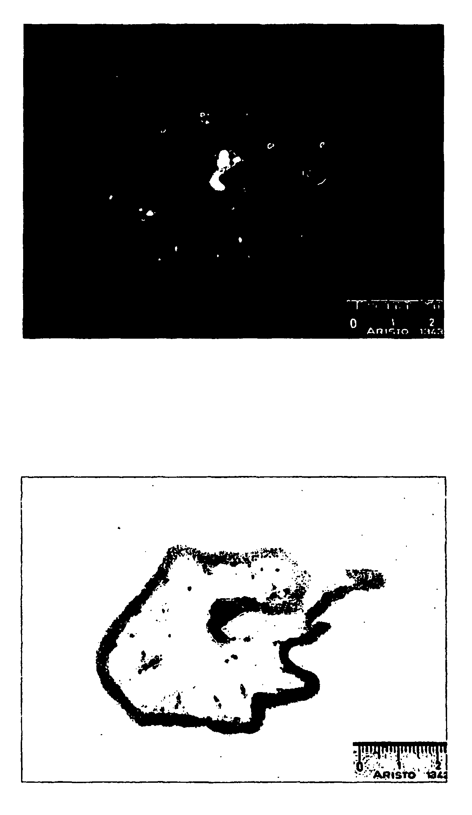

**Fig. 1**: The effectiveness of the dye conjugate in mesenterial Capan-1 micrometastasis 6 h after substance administration. The upper panel depicts the original image, while the lower panel depicts the inverted image. White and black dots or areas, respectively, show micrometastasis.

### Examples

### Example 10: Synthesis of Trisodium 3,3-dimethyl-2-{7-[3,3-dimethyl-5-sulfonato-1-(2-sulfonatoethyl)-3H-indolium-2-yl]4-(5-{[2-(2,5-dioxo-2,5-dihydro-1H-pyrrol-1-yl)ethyl]carbamoyl}-3-oxa-pentyl)hepta-2,4,6-trien-1-ylidene}-1-(2-sulfonatoethyl)-2,3-dihydro-1H-indole-5-sulfonate, internal salt (Formula XXVII)

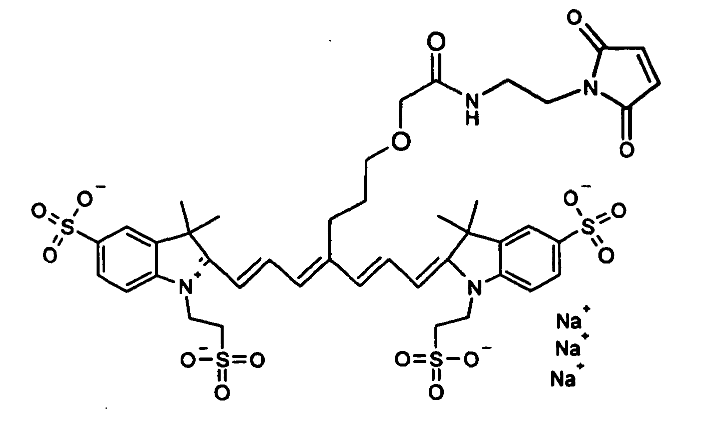

### a) 3-Oxa-6-(4-Pyridinyl)hexanoic acid-tert-butyl ester

A solution of 75 g (0.4 mol) of 3-(4-pyridinyl)-1-propanol in 400 ml of toluene/50 ml of THF is mixed with 10 g of tetrabutylammonium sulfate and 350 ml of 32% sodium hydroxide solution. Then, 123 g (0.68 mol) of bromoacetic acid-*tert*-butyl ester is added in drops and stirred for 18 hours at room temperature. The organic phase is separated, and the aqueous phase is extracted three times with diethyl ether. The combined organic phases are washed with NaCl solution, dried on sodium sulfate and concentrated by evaporation. After chromatographic purification (silica gel: mobile solvent hexane:ethyl acetate), 56 g of product (41% of theory) is obtained as a brownish oil.

### b) 3-[4-Oxa-5-(tert-butyloxycarbonyl)pentyl]glutaconaldehyde-dianilide-hydrobromide

A solution of 5.0 g (20 mmol) of 3-oxa-6-(4-pyridinyl)hexanoic acid-*tert*-butyl ester in 60 ml of diethyl ether is mixed with 3.7 g (40 mmol) of aniline and then at 0°C with a solution of 2.2 g (20 mmol) of bromocyanogen in 8 ml of diethyl ether. After 1 hour of stirring at 0°C, 50 ml of diethyl ether is mixed, and the red solid that is produced is filtered off, washed with ether and vacuum-dried. Yield: 8.5 g (85% of theory) of a violet solid.

### c) Trisodium 3,3-dimethyl-2-{7-[3,3-dimethyl-5-sulfonato-1-(2-sulfonatoethyl)-3H-indolium-2-yl]-4-(6-carboxy-4-oxahexyl)hepta-2,4,6-trien-1-ylidene}-1-(2-sulfonatoethyl)-2,3-dihydro-1H-indole-5-sulfonate, internal salt

A suspension of 3.0 g (6 mmol) of 3-[2-(*tert*-butyloxycarbonyl)ethyl]-glutaconaldehyde-dianilide-hydrobromide (Example 10b)) and 4.2 g (12 mmol) of 1-(2-sulfonatoethyl)-2,3,3-trimethyl-3H-indolenine-5-sulfonic acid (Example 1a)) in 50 ml of acetic acid anhydride and 10 ml of acetic acid is mixed with 2.5 g (30 mmol) of sodium acetate and stirred for 50 minutes at 120°C. After cooling, it is mixed with diethyl ether, the precipitated solid is filtered off, absorptively precipitated in acetone and dried under high vacuum. After chromatographic purification (RP-C 18-silica gel, mobile solvent water/methanol), removal of the methanol in a vacuum and freeze-drying, the title compound is immediately obtained. Yield: 2.3 g (41% of theory) of a blue lyophilizate.

### d) Trisodium 3,3-dimethyl-2-{7-[3,3-dimethyl-5-sulfonato-1-(2-sulfonatoethyl)-3H-indolium-2-yl]-4-(5-{[2-(2,5-dioxo-2,5-dihydro-1H-pyrrol-1-yl)ethyl]carbamoyl}-3-oxa-pentyl)hepta-2,4,6-trien-1-ylidene}-1-(2-sulfonatoethyl)-2,3-dihydro-1H-indole-5-sulfonate, internal salt

The synthesis is carried out analogously to Example 1c) from 1.0 g (1.1 mmol) of the title compound of Example 10c). Yield: 0.85 g (73% of theory) of a blue lyophilizate.

| | | | | | | | | | | | |
|---|---|---|---|---|---|---|---|---|---|---|---|
| Elementary analysis: | Cld.: | C | 47.54 | H | 4.46 | N | 5.28 | S | 12.09 | Na | 6.50 |
| | Fnd.: | C | 47.97 | H | 4.65 | N | 5.10 | S | 12.02 | Na | 6.68 |

### Example 20: Labeling of BSA (bovine serum albumin) with the title compound of Example 10

General instructions: A solution of 5 mg (0.074 µmol) of BSA *(Sigma Company)* in 5 ml of phosphate buffer (0.1 M Na₂HPO₄/NaH₂PO₄, pH 6.8) is mixed in each case with 0.74 µmol of the title compound of Example 10 (stock solutions of 0.5 mg/ml in PBS) and incubated for 30 minutes at 25°C. The purification of the conjugate is carried out by means of gel chromatography (column: Sephadex G50, diameter 1.5 cm, Pharmacia, eluant: PBS).

General instructions: A solution of 5 mg (0.074 µmol) of BSA (*Sigma Company)* in 5 ml of phosphate buffer (0.1 M borate buffer, pH 8.5) is mixed in each case with 1.10 µmol of the title compounds of Examples 17-19 (stock solutions of 0.5 mg/ml in PBS) and incubated for 5 hours at 25°C. The purification of the conjugate is carried out by means of gel chromatography (column: Sephadex G50, diameter 1.5 cm, Pharmacia, eluant: PBS).

### Example 22: Labeling of anti-ED-B-fibronectin scFv antibody AP39 (single chain fragment) with the title compound of Example 10.

AP39 is an scFv with a C-terminal cysteine and is present as a covalent S-S-dimer of the molar-mass of about 56,000 g/mol (Curr. Opin. Drug Discov. Devel.. 2002 Mar; 5(2): 204-13). By reduction of the disulfide bridges, two monomers with accessible SH groups are produced (molar mass 28,000 g/mol).

General instructions: 0.3 ml of a solution of AP39 in PBS (conc. 0.93 mg of dimer/ml) is mixed with 60 µl of a solution of tris(carboxyethyl)phosphine (TCEP) in PBS (2.8 mg/ml) and incubated under nitrogen for 1 hour at 25°C. Excess TCEP is separated by means of gel filtration on an NAP-5 column (eluant: PBS). The quantity of AP39-monomer obtained (OD₂₈₀ₙₘ = 1.4), determined by means of photometry, is 230-250 µg (volumes 0.5 - 0.6 ml). The solution is mixed with 0.03 µmol of the title compound of Example 10 (stock solutions of 0.5 mg/ml in PBS) and incubated for 30 minutes at 25°C. The conjugate is purified by gel chromatography on an NAP-5 column (eluant: PBS/10% glycerol). The immune reactivity of the conjugate solution is determined by means of affinity chromatography (ED-B-fibronectin resin) (J. Immunol. Meth. 1999, 231, 239). The immune reactivity of the conjugates obtained was >80% (AP39 before the conjugation >95%).

### Example 24: Imaging of micrometastasis in Capan-1 tumor bearing nude mice

Capan-1 tumor cells that were grown subconfluently in culture were trypsinized, centrifuged and resuspended in PBS. After staining with trypan blue and calculation of the cell concentration, the cell suspension was set at a concentration of 3x10⁷/ml. The cell suspension was cooled on ice until it was used. Three female nude mice (NMRI-nude, 24-25 g body weight) were anesthetized, and 30 µl (1x10⁶ cells/animal) of the cell suspension inoculated subcapsularly in the pancreas in each animal after abdominal incision. Each animal received 0.05 µmol/kg body weight (1.3 mg/kg body weight) of a substance comprising a cyanine dye according to Example 10, i.e. having a structure as depicted in formula XXVII, which had been conjugated to the EB-DF antibody AP39 according to the method of Example 22. This substance was administered intravenously at a time point that a clear tumor growth was palpable (about 12 to 14 weeks post tumor cell implantation). The animals were sacrificed 6 hours after substance administration and the mesenterium containing micrometastasis was imaged *ex vivo* for fluorescence signals using an intensified CCD camera. The fluorescence of the substance was excited by mesenterium irradiation with near-infrared ligth with 740 nm wavelength, which was produced with a laser diode (0.5 W output). The fluorescence images were stored digitally. Following, the size of micrometastasis were evaluated using a low magnification microscope (Stemi 2000-C, Fa. Carl Zeis). Fluorescence signals were received from micrometastasis in the range of 0.5 to 2.0 mm in diameter and from larger mesenterial metastasis and corresponds with the microscopic evaluation. The effectiveness of the dye conjugates is depicted in Figure 1 based on an example.

In the foregoing and in the following examples, all temperatures are set forth uncorrected in degrees Celsius, and all parts and percentages are by weight, unless otherwise indicated.

The preceding examples can be repeated with similar success by substituting the generically or specifically described reactants and/or operating conditions of this invention for those used in the preceding examples.

## Claims

1. Use of a conjugate of the general formula (I):
B-(D)ₙ (I),
wherein
B stands for an angiogenesis specific binding component,
D stands for a cyanine dye of formula 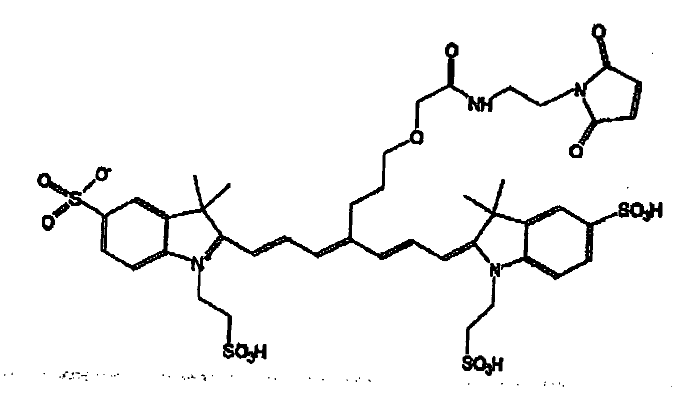 as well as salts and solvates of these compound,
n is 1 to 5,
for the production of a diagnostic for the diagnosis of micrometastasis or small proliferative lesions preceding the onset of angiogenesis, wherein, the small proliferative lesion and/or micrometastasis has a diameter of less than 10 mm wherein the angiogenesis specific binding component is antibody AP39, specific for ED-B fibromectin.

2. Use according of claim 1, wherein the early stage proliferative disease is selected from the group consisting of a small primary tumor, a precancerosis, a dysplasia, a metaplasia, psoriasis, psoriatic arthritis, rheumatoid arthritis, endometriosis and/or an ocular disease.

3. Use according of claim 1, wherein the micrometastasis and/or the small proliferative lesion is diagnosed prior, during and/or after a treatment procedure.

4. Use according of claim 1, wherein the micrometastasis and/or the small proliferative lesion has a diameter of less than 6 mm, preferably of less than 5 mm.

5. Use according to one of claims 1 to 4, wherein the micrometastasis and/or the small proliferative lesion has a diameter of less than 4 mm, preferably of less than 3 mm.

6. Use according to one of claims 1 to 5, wherein the micrometastasis and/or the small proliferative lesion has a diameter of between 2.0 to 0.2 mm.

7. Use according to one of claims 1 to 3, wherein the micrometastasis is an iatrogenic micrometastasis, a hematogenous micrometastasis, a cavitary micrometastasis, an intraluminal micrometastasis, a lymphatic metastasis, a local micrometastasis, and/or a regional micrometastasis.

8. Use according to claims 1 to 3, wherein the precancerosis is selected from the group consisting of precancerosis of the skin, in particular actinic keratosis, cutaneous horn, actinic cheilitis, tar keratosis, arsenic keratosis, x-ray keratosis, Bowen's disease, bowenoid papulosis, lentigo maligna, lichen sclerosus, and lichen ruber mucosae; precancerosis of the digestive tract, in particular erythroplakia, leukoplakia, Barrett's esophagus, Plummer-Vinson syndrome, crural ulcer, gastropathia hypertrophica gigantea, borderline carcinoma, neoplastic intestinal polyp, rectal polyp, porcelain gallbladder, gynaecological precancerosis, in particular carcinoma ductale in situ (CDIS), cervical intraepithelial neoplasia (CIN), leukoplakia, endometrial hyperplasia (grade III), vulvar dystrophy, vulvar intraepithelial neoplasia (VIN), hydatidiform mole; urologic precancerosis, in particular bladder papillomatosis, Queyrat's erythroplasia, testicular intraepithelial neoplasia (TIN), leukoplakia; carcinoma in situ (CIS); precancerosis caused by chronic inflammation, in particular pyoderma, osteomyelitis, acne conglobata, lupus vulgaris, and fistula.

9. Use according to claims 1 to 3, wherein the metaplasia is selected from the group consisting of agnogenic myeloid metaplasia, apocrine metaplasia, atypical metaplasia, autoparenchymatous metaplasia, connective tissue metaplasia, epithelial metaplasia, intestinal metaplasia, metaplastic anemia, metaplastic ossification, metaplastic polyps, myeloid metaplasia, primary myeloid metaplasia, secondary myeloid metaplasia, squamous metaplasia, squamous metaplasia of amnion, symptomatic myeloid metaplasia and regenerative metaplasia.

10. Use according to claims 1 to 3, wherein the dysplasia is selected from the group consisting of anhidrotic ectodermal dysplasia, anterofacial dysplasia, asphyxiating thoracic dysplasia, atriodigital dysplasia, bronchopulmonary dysplasia, cerebral dysplasia, cervical dysplasia, chondroectodermal dysplasia, cleidocranial dysplasia, congenital ectodermal dysplasia, craniodiaphysial dysplasia, craniocarpotarsal dysplasia, craniometaphysial dysplasia, dentin dysplasia, diaphysial dysplasia, ectodermal dysplasia, enamel dysplasia, encephalo-ophtalmic dysplasia, dysplasia epiphysialis heminelia, dysplasia epiphysialis multiplex, dysplasia epiphysalis punctata, epithelial dysplasia, faciodigitogenital dysplasia, familial fibrous dysplasia of jaws, familial white folded dysplasia, fibromuscular dysplasia, fibrous dysplasia of bone, florid osseous dysplasia, hereditary renal-retinal dysplasia hidrotic ectodermal dysplasia, hypohidrotic ectodermal dysplasia, lymphopenic thymic dysplasia, mammary dysplasia, mandibulofacial dysplasia, metaphysical dysplasia, Mondini dysplasia, monostotic fibrous dysplasia, mucoepithelial dysplasia, multiple epiphysial dysplasia, oculoauriculovertebral dysplasia, oculodentodigital dysplasia, oculovertebral dysplasia, odontogenic dysplasia, ophthalmomandibulomelic dysplasia, periapical cemental dysplasia, polyostotic fibrous dysplasia, pseudoachondroplastic spondyloepiphysial dysplasia, retinal dysplasia, septo-optic dysplasia, spondyloepiphysial dysplasia, and ventriculoradial dysplasia.

11. Use according to claims 1 to 3, wherein the endometriosis comprises hematogenous cell clusters, cavitary cell clusters, intraluminal cell clusters, lymphatic cell clusters, local cell clusters and/or regional cell clusters.

12. Use according to claims 1 to 3, wherein the ocular disease is selected from the group consisting of trachoma, retinopathy of prematurity, diabetic retinopathy, neovascular glaucoma and age-related macular degeneration.

## Patentansprüche

1. Verwendung eines Konjugats der allgemeinen Formel (I) :
B- (D) ₙ (I) ,
wobei
B für eine Angiogenese-spezifische Bindungskomponente steht,
D für einen Cyaninfarbstoff der Formel 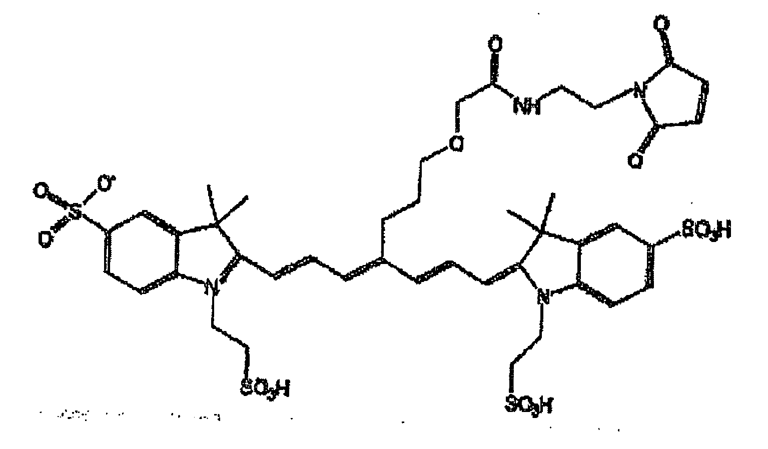 steht, sowie Salze und Solvate dieser Verbindung,
n 1 bis 5 beträgt,
für die Erzeugung eines Diagnostikums für die Diagnose von Mikrometastasen oder kleinen, proliferativen Läsionen, die der Entstehung der Angiogenese vorausgehen, wobei die kleine proliferative Läsion und/oder die Mikrometastase einen Durchmesser von weniger als 10 mm aufweist, wobei die Angiogenese-spezifische Bindungskomponente der für ED-B-Fibronektin spezifische AP39-Antikörper ist.

2. Verwendung nach Anspruch 1, wobei das Frühstadium der proliferativen Erkrankung aus der Gruppe ausgewählt ist, die aus einem kleinen Primärtumor, einer Präkanzerose, einer Dysplasie, einer Metaplasie, Psoriasis, psoriatischer Arthritis, rheumatoider Arthritis, Endometriose und/oder einer Augenkrankheit besteht.

3. Verwendung nach Anspruch 1, wobei die Mikrometastase und/oder die kleine proliferative Läsion vor, während und/oder nach einem Behandlungsablauf diagnostiziert wird.

4. Verwendung nach Anspruch 1, wobei die Mikrometastase und/oder die kleine proliferative Läsion einen Durchmesser von weniger als 6 mm, vorzugsweise von weniger als 5 mm aufweist.

5. Verwendung nach einem der Ansprüche 1 bis 4, wobei die Mikrometastase und/oder die kleine proliferative Läsion einen Durchmesser von weniger als 4 mm, vorzugsweise von weniger als 3 mm aufweist.

6. Verwendung nach einem der Ansprüche 1 bis 5, wobei die Mikrometastase und/oder die kleine proliferative Läsion einen Durchmesser zwischen 2,0 und 0,2 mm aufweist.

7. Verwendung nach einem der Ansprüche 1 bis 3, wobei die Mikrometastase eine iatrogene Mikrometastase, eine hämatogene Mikrometastase, eine kavitäre Mikrometastase, eine intraluminale Mikrometastase, eine lymphatische Metastase, eine lokale Mikrometastase, und/oder eine regionale Mikrometastase ist.

8. Verwendung nach einem der Ansprüche 1 bis 3, wobei die Präkanzerose aus der Gruppe ausgewählt ist, die aus Folgendem besteht: Präkanzerose der Haut, insbesondere aktinische Keratose, Hauthorn, aktinische Cheilitis, Teerkeratose, Arsenkeratose, Röntgenstrahlenkeratose, Morbus Bowen, bowenoide Papulose, Lentigo maligna, Lichen sclerosus und Lichen ruber mucosae; Präkanzerose des Verdauungstrakts, insbesondere Erythroplakie, Leukoplakie, Barrett-Ösophagus, Plummer-Vinson Syndrom, Ulcus cruris, Gastrophathia hypertrophica gigantea, Borderline-Karzinom, neoplastischer intestinaler Polyp, rektaler Polyp, Porzellangallenblase; gynäkologische Präkanzerosen, insbesondere duktales Carcinoma in situ (CDIS), zervikale intraepitheliale Neoplasie (CIN), Leukoplakie, Endometriumhyperplasie (Grad III), Vulvadystrophie, vulväre intraepitheliale Neoplasie (VIN), hydatiforme Mole; urologische Präkanzerosen, insbesondere Blasenpapillomatose, Queyrat-Erythroplasie, testikuläre intraepitheliale Neoplasie (TIN), Leukoplakie; Carcinoma in situ (CIS); durch chronische Entzündung bedingte Präkanzerose, insbesondere Pyoderma, Osteomyelitis, Acne conglobata, Lupus vulgaris und Fistel.

9. Verwendung nach Ansprüchen 1 bis 3, wobei die Metaplasie aus der Gruppe ausgewählt ist, die aus Folgendem besteht: agnogenische myeloische Metaplasie, apokrine Metaplasie, atypische Metaplasie, auto-parenchymatöse Metaplasie, Bindegewebsmetaplasie, epitheliale Metaplasie, intestinale Metaplasie, metaplastische Anämie, metaplastische Ossifikation, metaplastische Polypen, myeloide Metaplasie, primär myeloide Metaplasie, sekundär myeloide Metaplasie, squamöse Metaplasie, squamöse Metaplasie des Amnion, symptomatische myeloide Metaplasie und regenerative Metaplasie.

10. Verwendung nach Ansprüchen 1 bis 3, wobei die Dysplasie aus der Gruppe ausgewählt ist, die aus Folgendem besteht: anhidrotische ektodermale Dysplasie, antero-faziale Dysplasie, asphyktische thorakale Dysplasie, atrio-digitale Dysplasie, bronchopulmonale Dysplasie, zerebrale Dysplasie, zervikale Dysplasie, chondro-ektodermale Dysplasie, cleido-craniale Dysplasie, kongenitale ektodermale Dysplasie, cranio-diaphysäre Dysplasie, cranio-carpo-tarsale Dysplasie, craniometaphysäre Dysplasie, Dentindysplasie, diaphysäre Dysplasie, ektodermale Dysplasie, Zahnschmelzdysplasie, enzephalo-ophtalmische Dysplasie, Dysplasia epiphysialis heminelia, Dysplasia epiphysialis multiplex, Dysplasia epiphysialis punctata, epitheliale Dysplasie, facio-digito-genitale Dysplasie, familiäre fibröse Kieferdysplasie, familiärer weißer Schleimhaut-Naevus, fibromuskuläre Dysplasie, fibröse Knochendysplasie, floride Knochendysplasie, angeborene renal-retinale Dysplasie, hidrotische ektodermale Dysplasie, hypohidrotische ektodermale Dysplasie, lymphopenische Thymusdysplasie, Brustdysplasie, mandibulo-faziale Dysplasie, metaphysäre Dysplasie, Mondini-Dysplasie, monostotische fibröse Dysplasie, muko-epitheliale Dysplasie, multiple epiphysäre Dysplasie, okuloaurikulo-vertebrale Dysplasie, okulo-dentodigitale Dysplasie, okulo-vertebrale Dysplasie, odontogene Dysplasie, ophthalmo-mandibuläre Dysplasie, periapikale Zementdysplasie, polyostotische fibröse Dysplasie, pseudoachondroplastische spondylo-epiphysäre Dysplasie, retinale Dysplasie, septo-optische Dysplasie, spondylo-epiphysäre Dysplasie, und ventrikuloradiale Dysplasie.

11. Verwendung nach Ansprüchen 1 bis 3, wobei die Endometriose hämatogene Zellcluster, lokale Zellcluster und/oder regionale Zellcluster umfasst.

12. Verwendung nach Ansprüchen 1 bis 3, wobei die Augenerkrankung aus einer Gruppe ausgewählt ist, die aus Trachom, Retinopathia praematurorum, diabetischer Retinopathie, neovaskulärem Glaukom und altersbedingter Makuladegenration besteht.

## Revendications

1. Utilisation d'un conjugué de formule générale (I) :
B- (D) ₙ (I),
où
B représente un composé de liaison spécifique de l'angiogenèse,
D représente un colorant cyanine de formule 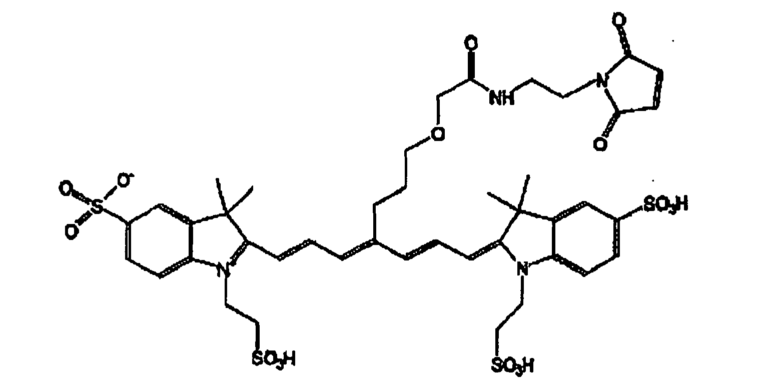 ainsi que des sels et des solvates de ce composé,
n représente 1 à 5,
pour la production d'un agent diagnostique destiné au diagnostic de micrométastases ou de petites lésions prolifératives précédent le début d'une angiogenèse, où la petite lésion proliférative et/ou la micrométastase présente un diamètre inférieur à 10 mm où le composant de liaison spécifique de l'angiogenèse est l'anticorps AP39, spécifique de la ED-B fibronectine.

2. Utilisation selon la revendication 1, où la maladie proliférative au stade précoce est choisie dans le groupe constitué par une petite tumeur primaire, un précancer, une dysplasie, une métaplasie, le psoriasis, l'arthrite psoriasique, l'arthrite rhumatoïde, l'endométriose et/ou une maladie oculaire.

3. Utilisation selon la revendication 1, où la micrométastase et/ou la petite lésion proliférative est diagnostiquée avant, pendant et/ou après une procédure de traitement.

4. Utilisation selon la revendication 1, où la micrométastase et/ou la petite lésion proliférative présente un diamètre inférieur à 6 mm, de préférence inférieur à 5 mm.

5. Utilisation selon l'une quelconque des revendications 1 à 4, où la micrométastase et/ou la petite lésion proliférative présente un diamètre inférieur à 4 mm, de préférence inférieur à 3 mm.

6. Utilisation selon l'une quelconque des revendications 1 à 5, où la micrométastase et/ou la petite lésion proliférative présente un diamètre entre 2,0 et 0,2 mm.

7. Utilisation selon l'une quelconque des revendications 1 à 3, où la micrométastase est une micrométastase iatrogénique, une micrométastase hématogène, une micrométastase cavitaire, une micrométastase intraluminale, une micrométastase lymphatique, une micrométastase locale, et/ou une micrométastase régionale.

8. Utilisation selon les revendications 1 à 3, où le précancer est choisi dans le groupe formé par un précancer de la peau, en particulier la kératose actinique, le papillome corné, la chéilite actinique, la kératose du goudron, la kératose arsénique, la kératose de rayons X, la maladie de Bowen, la papulose bowénoïde, le lentigo malin, le lichen scléreux, et le lichen ruber de la muqueuse ;un précancer du tractus digestif, en particulier l'érythroplasie, la leucoplasie, l'oesophage de Barrett, le syndrome de Plummer-Vinson, l'ulcère crural, la gastrite hypertrophique géante, le carcinome borderline, un polype intestinal néoplasique, un polype rectal, la vésicule de porcelaine ; un précancer gynécologique, en particulier le carcinome canalaire in situ (CDIS), la néoplasie intra-épithéliale cervicale (CIN), la leucoplasie, l'hyperplasie endométriale (grade III), la dystrophie vulvaire, la néoplasie intra-épithéliale vulvaire (VIN), le môle hydatidiforme ; un précancer urologique, en particulier la papillomatose de la vessie, l'érythroplasie de Queyrat, la néoplasie intra-épithéliale testiculaire (TIN), la leucoplasie ; le carcinome in situ (CIS) ; le précancer provoqué par une inflammation chronique, en particulier le pyoderma, l'ostéomyélite, l'acné conglobata, le lupus vulgaire, et les fistules.

9. Utilisation selon les revendications 1 à 3, où la métaplasie est choisie dans le groupe constitué par la métaplasie myéloïde agnogène, la métaplasie apocrine, la métaplasie atypique, la métaplasie autoparenchymateuse, la métaplasie du tissu connectif, la métaplasie épithéliale ; la métaplasie intestinale, l'anémie métaplasique, l'ossification métaplasique, les polypes métaplasiques, la métaplasie myéloïde, la métaplasie myéloïde primaire, la métaplasie myéloïde secondaire, la métaplasie squameuse, la métaplasie squameuse de l'amnios, la métaplasie myéloïde symptomatique et la métaplasie régénérative.

10. Utilisation selon les revendications 1 à 3, où la dysplasie est choisie dans le groupe formé par la dysplasie anhidrotique ectodermique, la dysplasie antérofaciale, la dysplasie thoracique asphyxiante, la dysplasie atrio-digitale, la dysplasie bronchopulmonaire, la dysplasie cérébrale, la dysplasie cervicale, la dysplasie chondroectodermique, la dysplasie cléido-crânienne, la dysplasie ectodermique congénitale, la dysplasie craniodiaphysaire, la dysplasie craniocarpotarsale, la dysplasie craniométaphysaire, la dysplasie de la dentine, la dysplasie diaphysaire, la dysplasie ectodermique, la dysplasie de l'émail, la dysplasie encéphalo-ophtalmique, la dysplasie épiphysaire hémimélique, la dysplasie épiphysaire multiple, la dysplasie épiphysaire ponctuée, la dysplasie épithéliale, la dysplasie faciodigitogénitale, la dysplasie fibreuse familiale des mâchoires, le nevus blanc spongieux, la dysplasie fibromusculaire, la dysplasie fibreuse de l'os, la dysplasie osseuse floride, la dysplasie héréditaire rénale-rétinale, la dysplasie ectodermique hidrotique, la dysplasie hypohidrotique ectodermique, la dysplasie lymphopénique thymique, la dysplasie mammaire, la dysplasie mandibulofaciale, la dysplasie métaphysaire, la dysplasie de Mondini, la dysplasie monostotique fibreuse, la dysplasie mucoépithéliale, la dysplasie épiphysaire multiple, la dysplasie oculoauriculovertébrale, la dysplasie oculodentodigitale, la dysplasie oculovertébrale, la dysplasie odontogénique, la dysplasie ophtalmomandibulomélique, la dysplasie périapicale cémentale, la dysplasie polyostotique fibreuse, la dysplasie pseudoachondroplasique spondyloépiphysaire, la dysplasie rétinale, la dysplasie septo-optique, la dysplasie spondyloépiphysaire, et la dysplasie ventriculoradiale.

11. Utilisation selon les revendications 1 à 3, où l'endométriose comprend les clusters cellulaires hématogènes, les clusters cellulaires cavitaires, les clusters cellulaires intraluminaux, les clusters cellulaires lymphatiques, les clusters cellulaires locaux et/ou les clusters cellulaires régionaux.

12. Utilisation selon les revendications 1 à 3, où la maladie oculaire est choisie dans le groupe constitué par le trachome, la rétinopathie de prématurité, la rétinopathie diabétique, le glaucome néovasculaire et la dégénérescence maculaire associée à l'âge.
